# EUROPEAN PATENT APPLICATION

(11) **EP 4 706 641 A1**
(43) Date of publication of application: **11.03.2026**
(21) Application number: 25200811.5
(22) Date of filing: 08.09.2025
(51) Int. Cl.: A61K 9/48, A61K 31/495

(54) **A CAPSULE FORMULATION COMPRISING CARIPRAZINE HYDROCHLORIDE**

(30) Priority: 10.09.2024 TR 2024011969
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); PEHLIVAN AKALIN, Nur, Istanbul (TR); ARMUT, Merve, Istanbul (TR); OZTAS, Bora, Istanbul (TR)
(74) Representative: Genç Ilhan, Oznur

(57) **Abstract**

The present invention relates to a capsule formulation comprising cariprazine hydrochloride and at least one filler, wherein the filler is sodium starch glycolate and its amount is between 85.0% and 99.0% in the total formulation. The present invention also relates to a simple, rapid, cost effective, time-saving and industrially convenient method of preparing the formulation.

## Description

### Field of the Invention

The present invention relates to a capsule formulation comprising cariprazine hydrochloride and at least one filler, wherein the filler is sodium starch glycolate and its amount is between 85.0% and 99.0% in the total formulation. The present invention also relates to a simple, rapid, cost effective, time-saving and industrially convenient method of preparing the formulation.

### Background of the Invention

Schizophrenia is a lifelong disabling psychiatric disorder with a reported worldwide prevalence of about 1%, including 3.2 million Americans. The disorder usually manifests during adolescence or in young adulthood; the cardinal symptoms fall into three domains - positive symptoms, such as delusions and hallucinations, negative symptoms, such as lack of drive and social withdrawal, and cognitive symptoms, such as problems with attention and memory. These lead to social and occupational dysfunction, which inevitably have a profound effect on the family and the place of the affected individual in wider society. In addition to psychiatric symptoms, patients with schizophrenia are at greater risk for medical comorbidities than the general population.

Drug treatment with dopamine antagonists is the cornerstone of schizophrenia management both during the acute as well as the residual phase. Current guidelines recommend atypical antipsychotics, including risperidone, olanzapine, quetiapine, ziprasidone, and aripiprazole, as first-line treatment for schizophrenia. These drugs can be uniformly characterized by their dual mode of action: in addition to antagonism of the dopamine D2receptor, they are also potent inhibitors at the serotonin 5-HT2A receptor.

Cariprazine hydrochloride is a novel atypical antipsychotic that has antagonistic effect on dopamine D3 receptor, dopamine D2 receptor and 5-hydroxytryptamine 2B receptor. It can be used for treating schizophrenia and bipolar I disorder.

The chemical name of cariprazine hydrochloride is 3-[4-[2-[4-(2,3-dichlorophenyl)piperazin-1-yl]ethyl]cyclohexyl]-1,1-dimethylurea;hydrochloride and its chemical structure is shown in the Formula I:

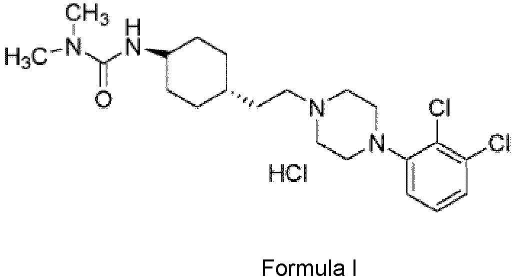

Cariprazine hydrochloride capsules are currently available on the market. This product is an oral preparation that should be taken daily to maintain its plasma concentration.

The patent CA2715760C disclosed is a solid preparation for oral administration, which comprises cariprazine hydrochloride, in which lactose is used as the main excipient, and which enables the stable storage of cariprazine hydrochloride contained therein without the need of adding cyclodextrin.

The medical oral preparation of cariprazine disclosed in patent CN106692149 is prepared from cariprazine, a filler, a disintegrant, a lubricant, a flow aid and an adhesive. The particle size of 90 percent of cariprazine with ultrafine particle size is 25 micrometers, and the particle size of 99 percent of cariprazine is less than 30 micrometers.

Formulations of cariprazine hydrochloride are known in the prior art. However, a formulation that simultaneously provides flowability, content uniformity and ease of capsule filling was not found. Therefore, a better formulation is still needed.

In our formulation, we used a high percentage of sodium starch glycolate with a bulk density greater than 0.35 g/cm3 to create a capsule formulation with better flowability, content uniformity and ease of capsule filling.

### Detailed Description of the Invention

The main object of the present invention is to provide a capsule formulation containing cariprazine hydrochloride, characterized by excellent pharmacotechnical properties such as flowability, content uniformity and ease of capsule filling.

Another object of the present invention is to a capsule formulation comprising cariprazine hydrochloride providing the desired dissolution profile and stability.

Another object of the present invention is to provide a method for a capsule formulation comprising cariprazine hydrochloride prepared by simple, easy, time-saving and fast manufacturing methods.

Cariprazine hydrochloride ratio is used very low amount in formulations. Therefore, the use of filler(s) in the formulation is quite high amount. This causes some problems such as flowability, content uniformity and capsule filling problems.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed. The bulk density is expressed in grams per milliliter (g/mL) although the international unit is kilogram per cubic meter (1 g/mL=1000 kg/m³). It may also be expressed in grams per cubic centimeter (g/cm³).

When substances with very low bulk density are used, filling into capsules of size 3 and smaller size is a problem in direct mixing processes. Since the formulation contains a high proportion of fillers, using a material with a bulk density greater than 0.35 g/cm³ facilitates the filling process into capsules of size 3 and smaller. For this reason, we used sodium starch glycolate with a bulk density greater than 0.35 g/cm³. This physical property of the sodium starch glycolate is very important for making the capsule that can be filled into suitable sized capsules for human consumption. In particular, we have seen that the use of fillers with a bulk density greater than 0.35 g/cm3 in cariprazine formulations overcomes the problems of flowability, content uniformity and capsule filling. In fact, the best results were obtained when sodium starch glycolate was used in the range of 85.0% to 99.0% of the total formulation.

Bulk density is measured by gravimetric determination of a measured volume.

According to one embodiment, a capsule formulation comprising cariprazine hydrochloride and at least one filler, wherein the filler is sodium starch glycolate and its amount is between 85.0% and 99.0% in the total formulation.

According to one embodiment of the present invention, the amount of cariprazine hydrochloride is between 0.1% and %10.0 by weight in the total formulation.

According to this embodiment of the present invention, the capsule formulation further comprises at least one lubricant.

Suitable lubricants are selected from the group comprising magnesium stearate, anhydrous colloidal silicon dioxide, sodium stearyl fumarate, magnesium oxide, silicone dioxide, talc, polyethylene glycol, stearic acid, aluminum silicate, magnesium silicate, colloidal silica, or mixtures thereof.

According to an embodiment of the present invention, the lubricant is magnesium stearate.

According to this embodiment of the present invention, the amount of lubricant is between 0.01% and 5.0% by weight of the total formulation.

As used here in, 'particle size' means the cumulative volume size distribution as tested by any conventionally accepted method such as the laser diffraction method (i.e. malvern analysis). The term d (0.9) means, the size at which 90% by volume of the particles are finer. The term d (0.5) means, the size at which 50% by volume of the particles are finer.

According to one embodiment, cariprazine hydrochloride has a d (0.9) particle size between 30 µm and 2 µm, between 25 µm and 3 µm, between 20 µm and 3 µm, between 15 µm and 4 µm, between 10 µm and 1 µm. The use of cariprazine hydrochloride at particle sizes d (0.9) in these ranges provided the desired dissolution profile and stability.

According to this embodiment of the present invention, the capsule formulation comprises;
- Cariprazine hydrochloride
- Sodium starch glycolate
- A Lubricant

According to this embodiment of the present invention, the capsule formulation comprises;
- Cariprazine hydrochloride
- Sodium starch glycolate
- Magnesium Stearate

According to this embodiment of the invention, a method for preparing capsule formulations comprises the following steps:
a) Cariprazine and a small portion of sodium starch glycolate sieved together,
b) Sieving the entire remainder of the sodium starch glycolate through a sieve,
c) Mixing the mixtures in steps (a) and (b) by geometric mixing method,
d) Sieving the final mixture through a sieve,
e) Sieve the magnesium stearate and add to the mixture in step (d) and mix,
f) Filling the mixture into capsules.

### Example 1; Capsule formulation

| **Ingredients** | **% by weight** | **% by weight** | **% by weight** | **% by weight** | **% by weight** |
|---|---|---|---|---|---|
| Cariprazine Hydrochloride (equivalent to 3.0 mg cariprazine) | 1.21 | 2.41 | 3.62 | 4.82 | 0.1-10 |
| Sodium Starch Glycolate | 98.05 | 96.85 | 95.64 | 94.44 | 85-99 |
| Magnesium Stearate | 0.74 | 0.74 | 0.74 | 0.74 | 0.01-5 |
| **Total In-Capsule Weight** | **100** | **100** | **100** | **100** | **100** |
| **NO _3 Hard Gelatin Capsule** | | | | | |

A process for example 1;
a) Weighing of raw materials,
b) Cariprazine and a small portion of sodium starch glycolate sieved together,
c) Sieving the entire remainder of the sodium starch glycolate through a sieve,
d) Mixing the mixtures in steps (b) and (c) by geometric mixing method,
e) Sieving the final mixture through a sieve,
f) Sieve the magnesium stearate and add to the mixture in step (e) and mix,
g) Filling the prepared mixture into capsules with an in-capsule weight of 135 mg.

### Example 2;

| **Ingredients** | **% by weight** |
|---|---|
| Cariprazine Hydrochloride | 0.1-10 |
| A Filler | 85-99 |
| A Lubricant | 0.01-5 |
| **Total In-Capsule Weight** | **100** |

## Claims

1. A capsule formulation comprising cariprazine hydrochloride and at least one filler, wherein the filler is sodium starch glycolate and its amount is between 88.0% and 99.0% in the total formulation.

2. The capsule formulation according to claim 1, wherein the amount of cariprazine hydrochloride is between 0.1% and %10.0 by weight in the total formulation.

3. The capsule formulation according to claim 1, wherein the capsule formulation further comprises at least one lubricant.

4. The capsule formulation according to claim 1, wherein lubricants are selected from the group comprising magnesium stearate, anhydrous colloidal silicon dioxide, sodium stearyl fumarate, magnesium oxide, silicone dioxide, talc, polyethylene glycol, stearic acid, aluminum silicate, magnesium silicate, colloidal silica, or mixtures thereof.

5. The capsule formulation according to claim 1, wherein the lubricant is magnesium stearate.

6. The capsule formulation according to claim 1, wherein the amount of lubricant is between 0.01% and 5.0% by weight of the total formulation.

7. The capsule formulation according to claim 1, wherein cariprazine hydrochloride has a d (0.9) particle size between 30 µm and 2 µm, between 25 µm and 3 µm, between 20 µm and 3 µm, between 15 µm and 4 µm, between 10 µm and 1 µm.

8. The capsule formulation according to any preceding claim, wherein the capsule formulation comprises;
• Cariprazine hydrochloride
• Sodium starch glycolate
• A Lubricant

9. The capsule formulation according to any preceding claim, wherein the capsule formulation comprises;
• Cariprazine hydrochloride
• Sodium starch glycolate
• Magnesium Stearate

10. The capsule formulation according to any preceding claim, wherein a method for preparing capsule formulation comprises the following steps:
a) Cariprazine and a small portion of sodium starch glycolate sieved together,
b) Sieving the entire remainder of the sodium starch glycolate through a sieve,
c) Mixing the mixtures in steps (a) and (b) by geometric mixing method,
d) Sieving the final mixture through a sieve,
e) Sieve the magnesium stearate and add to the mixture in step (d) and mix, 0f) Filling the mixture into capsules.
